(19) Europäisches Patentamt

European Patent Office

Office européen des brevets

(11) EP 0 795 322 B1

(12) **FASCICULE DE BREVET EUROPEEN**

(45) Date de publication et mention
de la délivrance du brevet:
**28.10.1998 Bulletin 1998/44**

(51) Int. Cl.$^6$: **A61K 7/48**, C08L 5/14,
A61K 47/36

(21) Numéro de dépôt: **97400241.2**

(22) Date de dépôt: **03.02.1997**

(54) **Composition épaissie par un alkyléther de polysaccharide**

Mit einem Alkylether-Polysaccharid verdickte Zusammensetzung

Composition thickened with an alkylether of a polysaccharide

(84) Etats contractants désignés:
**DE ES FR GB IT**

(30) Priorité: **12.03.1996 FR 9603097**

(43) Date de publication de la demande:
**17.09.1997 Bulletin 1997/38**

(73) Titulaire: **L'OREAL**
**75008 Paris (FR)**

(72) Inventeurs:
• **Arnaud, Pascal**
**94000 Creteil (FR)**
• **Simon, Pascal**
**94400 Vitry Sur Seine (FR)**

(74) Mandataire: **Lhoste, Catherine**
**L'OREAL,**
**D.P.I.,**
**90 rue du Général Roguet**
**92583 Clichy Cédex (FR)**

(56) Documents cités:
**EP-A- 0 281 360**    **EP-A- 0 682 936**
**EP-A- 0 708 114**

• **RESEARCH DISCLOSURE, no. 37807, Octobre**
**1995, page 642 XP002018143 MAJEWICZ ET AL.:**
**"oil-based cosmetic and therapeutic**
**compositions containing ethylguar"**
• **RESEARCH DISCLOSURE, no. 38413, 10 Avril**
**1996, pages 235-236, XP002033268 MAJEWICZ**
**ET AL.: "ethyl galactomannan film properties for**
**use in personal care applications"**

**Description**

La présente invention se rapporte à composition épaissie contenant un nouvel épaississant de phase grasse, destinée en particulier aux domaines cosmétique et/ou dermatologique. L'invention se rapporte aussi à une utilisation de cette composition pour le traitement, le maquillage et/ou le soin de la peau et/ou des muqueuses d'humains.

Dans les produits cosmétiques ou dermatologiques, il est courant de trouver une phase grasse épaissie ; ceci est notamment le cas dans les onguents, les gels de soins ou gommants anhydres, les compositions solides comme les déodorants, les baumes et les rouges à lèvres. L'épaississement des huiles permet en particulier de limiter l'exsudation des huiles des compositions solides et, en plus, dans les rouges à lèvres la migration du film rouge sur les lèvres.

Pour remédier à ces problèmes, on a habituellement recours à des cires ou des charges. Malheureusement, ces cires et charges ont tendance à matifier la composition, ce qui n'est pas toujours souhaitable. En particulier, les femmes sont de plus en plus à la recherche d'un rouge à lèvres brillant.

Il est, par ailleurs connu d'épaissir les huiles avec des épaississants polymériques. Malheureusement, les épaississants d'huiles connus doivent être utiliser en grande quantité pour obtenir un gel de viscosité élevée, à savoir supérieur à 1,3 Pa.s. Or, une trop grande quantité de épaississant confère à la composition, lorsque cette dernière est destinée au domaine cosmétique, des propriétés insuffisantes, notamment un toucher collant et un manque de glissant, ces inconvénients pouvant être très gênants, voire rédhibitoires.

De manière surprenante et inattendue, la demanderesse a découvert qu'il était possible d'épaissir fortement certaines phases grasses et d'obtenir des compositions cosmétiques stables sous forme de gel transparent présentant des propriétés cosmétiques satisfaisantes, grâce à un épaississant particulier.

La présente invention a donc pour objet une composition contenant une phase grasse et un épaississant de ladite phase grasse, cet épaississant comprenant au moins un alkyléther de polysaccharide formé de motifs comportant au moins deux cycles osidiques différents, chaque motif comportant au moins un groupe hydroxyle substitué par une chaîne alkyle hydrocarbonée saturée, et la phase grasse présentant les paramètres de solubilité $\delta_D$ et $\delta_a$ selon l'espace de solubilité de Hansen satisfaisant aux conditions suivantes :

$$15,00 \ (J/cm^3)^{1/2} \leq \delta_D \leq 18,00 \ (J/cm^3)^{1/2}$$

$$6,50 \ (J/cm^3)^{1/2} \leq \delta_a \leq 10,00 \ (J/cm^3)^{1/2}.$$

Grâce à cet épaississant particulier et à cette sélection de phase grasse, la composition selon l'invention est transparente et présente une viscosité supérieure à 1,3 Pa.s (1300 cps) pour un pourcentage en poids de 4%. Grâce à cet épaississant et à cette sélection de corps gras, il est possible d'obtenir, en association avec d'autres ingrédients, une composition solide en forme de stick présentant des propriétés cosmétiques améliorées par rapport aux compositions épaissies de l'art antérieur. En particulier, il est possible d'obtenir un bâton de rouge à lèvres doux à l'application ne migrant pas sur la peau contiguë aux lèvres et ayant un aspect brillant.

Bien que l'invention soit particulièrement bien adaptée au domaine cosmétique, elle s'applique à tout domaine nécessitant d'obtenir des compositions épaisses voire des compositions solides et notamment dans les domaines agroalimentaire, vétérinaire, dermatologique, pharmaceutique, du bois (stick de pigments et de cire pour la restauration de meubles).

De préférence, $16,00 \ (J/cm^3)^{1/2} \leq \delta_D \leq 18,00 \ (J/cm^3)^{1/2}$.

L'invention a aussi pour objet l'utilisation d'un alkyléther de polysaccharide formé de motifs comportant au moins deux cycles osidiques différents, chaque motif comportant au moins un groupe hydroxyle substitué par une chaîne alkyle hydrocarbonée saturée, pour épaissir une phase grasse présentant les paramètres de solubilité $\delta_D$ et $\delta_a$ selon l'espace de solubilité de Hansen satisfaisant aux conditions suivantes :

$$15,00 \ (J/cm^3)^{1/2} \leq \delta_D \leq 18,00 \ (J/cm^3)^{1/2}$$

$$6,50 \ (J/cm^3)^{1/2} \leq \delta_a \leq 10,00 \ (J/cm^3)^{1/2}.$$

La définition des corps gras dans l'espace de solubilité tridimensionnel selon HANSEN est décrite dans l'article de C. M. HANSEN : "The three dimensionnal solubility parameters" J. Paint Technol. 39, 105 (1967).

Selon cet espace de Hansen :

- $\delta_D$ caractérise les forces de dispersion de LONDON issues de la formation de dipôles induits lors des chocs moléculaires ;
- $\delta_p$ caractérise les forces d'interactions de DEBYE entre dipôles permanents ;
- $\delta_h$ caractérise les forces d'interactions spécifiques (type liaisons hydrogène, acide/base, donneur/accepteur, etc...)

;

- $\delta_a$ est déterminé par l'équation :

$$\delta_a = (\delta_p{}^2 + \delta_h{}^2)^{\frac{1}{2}}$$

Les paramètres $\delta_p$, $\delta_h$, $\delta_D$ et $\delta_a$ sont exprimés en $(J/cm^3)^{\frac{1}{2}}$.

Dans la composition selon l'invention, on peut utiliser n'importe quel corps gras ou mélange de corps satisfaisant aux relations ci-dessus. Dans ce cas, les paramètres de solubilité du mélange sont déterminés à partir de ceux des corps gras pris séparément, selon les relations suivantes :

$$\delta_{Dmel} = \sum_i xi\, \delta_{Di} \; ; \; \delta_{pmel} = \sum_i xi\, \delta_{pi} \; et \; \delta_{hmel} = \sum_i xi\, \delta_{hi}$$

$$\delta_{amel} = (\delta^2{}_{pmel} + \delta^2{}_{hmel})^{1/2}$$

où xi représente la fraction volumique du corps gras i dans le mélange. Il est donc possible d'obtenir des compositions épaissies notamment sous forme de gel ayant une viscosité supérieure à 1,3 Pa.s avec des corps gras dont les paramètres de solubilité ne sont pas compris dans les relations précédentes. Il est à la portée de l'homme du métier de déterminer les quantités de chaque corps gras pour obtenir un mélange de corps gras satisfaisant aux relations ci-dessus.

Comme corps gras satisfaisant aux relations ci-dessus, on peut citer les triglycérides d'acide gras de 4 à 10 atomes de carbone comme les triglycérides des acides heptanoïque ($\delta_D$ = 16,62 et $\delta_a$ = 7,29), octanoïque ($\delta_D$ = 16,35 et $\delta_a$ = 6,87), et des acides caprique/caprylique ($\delta_D$ = 16,64 et $\delta_a$ = 6,69) ; des huiles de synthèse ayant de 12 à 26 atomes de carbone comme l'octyldodécanol ($\delta_D$ = 16,36 et $\delta_a$ = 7,69), le 2-butyloctanol ($\delta_D$ = 16,12 et $\delta_a$ = 9,78), le 2-hexyldécanol ($\delta_D$ = 16,26 et $\delta_a$ = 8,55), le 2-undécylpentadécanol ($\delta_D$ = 16,45 et $\delta_a$ = 6,79) ; les esters d'acides gras notamment de 16 à 22 atomes de carbone comme le dioctanoate de propylène glycol ($\delta_D$ = 16,07 et $\delta_a$ = 6,68), le diheptanoate de néopentylglycol ($\delta_D$ = 16,37 et $\delta_a$ = 6,69), le diisononanoate de diéthylèneglycol ($\delta_D$ = 16,20 et $\delta_a$ = 6,86) ; l'alcool oléïque ($\delta_D$ = 16,28 et $\delta_a$ = 8,17) ; les esters hydroxylés de synthèse comme l'isostéaryl lactate ($\delta_D$ = 16,36 et $\delta_a$ = 8,74), l'octylhydroxystéarate ($\delta_D$ = 16, 43 et $\delta_a$ = 7,89), l'hydroxystéarate d'octyldodécyle ($\delta_D$ = 16,54 et $\delta_a$ = 6,56), le diisostéarylmalate ($\delta_D$ = 16,61 et $\delta_a$ = 7,19), le citrate de triisocétyle ($\delta_D$ = 16,77 et $\delta_a$ = 6,83) ; les triglycérides hydroxylés comme l'huile de ricin ($\delta_D$ = 16,79 et $\delta_a$ = 9,09), ou encore un mélange d'un ou plusieurs de ces corps gras.

Comme corps gras ne satisfaisant pas aux relations de Hansen ci-dessus, pris isolément, on peut citer les silicones volatiles ou non, les huiles d'origine minérale comme l'huile de vaseline et de paraffine, les huiles d'origine végétale comme l'huile de jojoba, de sésame, de colza, de beurre de karité, les huiles de synthèse comme l'huile de Purcellin, le palmitate d'éthyl-2 hexyle, le stéarate d'octyl-2 dodécyle, l'érucate d'octyl-2 dodécyle, l'isostéarate d'isostéaryle, le benzoate d'octyl-2 dodecyle et l'isoparaffine (6,8-moles d'isobutylène) hydrogénée. Ces huiles ne satisfaisant pas aux relations ci-dessus sont utilisées à des concentrations de préférence ne dépassant pas 50 %. En revanche, les huiles satisfaisant aux relations de Hansen ci-dessus peuvent représenter jusqu'à 99% de la phase grasse.

Dans l'épaississant de l'invention, on entend par 《 chaîne alkyle hydrocarbonée saturée 》 une chaîne comportant de 1 à 24, de préférence de 1 à 10 et mieux de 1 à 3 atomes de carbone. En particulier, la chaîne alkyle est choisie parmi les chaînes méthyle, éthyle, n-propyle, isopropyle, n-butyle, isobutyle, tertiobutyle, n-pentyle.

Selon un mode de réalisation préféré de l'invention, l'alkyléther de polysaccharide a un poids moléculaire supérieur à 100 000, et de préférence supérieur à 200 000. Ce poids moléculaire peur aller jusqu'à 1 million. Cet alkyléther peut comporter de un à six et mieux de deux à quatre groupes hydroxyle par motif, substitués par une chaîne alkyle hydrocarbonée saturée.

Les cycles osidiques sont notamment choisis parmi le mannose, le galactose, le glucose, le furanose, le rhamnose, l'arabinose.

Selon un mode préféré de réalisation de l'invention, l'alkyléther de polysaccharide selon l'invention est un alkyléther d'une gomme et plus particulièrement d'une gomme globalement non ionique, c'est-à-dire comportant peu ou pas de groupe ionique. Comme gommes appropriées, on peut citer par exemple la gomme guar dont le motif comprend un galactose et un mannose, la gomme de caroube dont le motif comprend un galactose et un mannose, la gomme de karaya qui est un mélange complexe de rhamnose, galactose et acide galacturonique, la gomme adragante qui est un mélange complexe d'arabinose, galactose et acide galacturonique.

Selon un mode de réalisation préféré de l'invention, l'alkyléther de polysaccharide est un dérivé de gomme guar, et plus particulièrement la guar éthylée ayant un degré de substitution de 2 à 3 et notamment d'environ 2,5 telle que décrite dans le document RD 95378007 (octobre 1995).

La composition selon l'invention peut contenir par exemple une quantité d'alkyléther de polysaccharide allant de 0,5 à 10 %, et de préférence de 2 à 8 % du poids total de la composition.

La composition selon l'invention peut de plus comprendre tous les ingrédients classiquement utilisés dans les domaines concernés et plus spécialement dans les domaines cosmétique et dermatologique. Ces ingrédients sont en particuliers choisis parmi les conservateurs, les vitamines et autres actifs lipophiles (lanoline, filtre UVA) ou hydrophiles (hydratants comme la glycérine), les épaississants de phase aqueuse ou grasse différents de celui de l'invention, les parfums, les tensioactifs, l'eau, les antioxydants, les charges, les cires, et leurs mélanges. La composition selon l'invention peut contenir également des vésicules lipidiques de type ionique et/ou non ionique. Les quantités de ces différents ingrédients sont celles classiquement utilisées dans les domaines concernés et par exemple de 0,01 % à 20 % du poids total de la composition. La nature de ces ingrédients et leur proportion doit être compatibles avec l'obtention de compositions stables épaissies selon l'invention.

La composition de l'invention peut se présenter sous toutes les formes galéniques normalement utilisées pour une application topique ou orale, et notamment sous forme d'un gel huileux, d'une émulsion huile-dans-eau ou eau-dans-huile, d'une dispersion d'huile dans de l'eau grâce à des vésicules, les vésicules étant situées à l'interface huile/eau. Cette composition peut avoir l'aspect d'une crème, d'une pommade, d'une pâte, d'un onguent, d'un stick.

La composition selon l'invention peut être avantageusement utilisée pour le traitement, le maquillage ou le soin de la peau et/ou des muqueuses selon la nature des actifs utilisés. En particulier, la composition de l'invention peut être un bâton de rouge à lèvres, un baume à lèvres pouvant traiter les gerçures ou encore une composition à appliquer sur le film de rouge à lèvres notamment pour en augmenter sa brillance.

Aussi, l'invention a encore pour objet une utilisation cosmétique de la composition pour soigner et/ou maquiller les lèvres ainsi qu'une utilisation de cette composition pour la préparation d'un onguent destiné à traiter les lèvres. L'invention a aussi pour objet un procédé de traitement cosmétique et/ou dermatologique des lèvres, consistant à appliquer sur les lèvres la composition définie ci-dessus.

Les exemples de compositions ci-après sont des exemples hypothétiques mais tout-à-fait réalisables par l'homme du métier ; ils sont donnés à titre d'illustration et sans caractère limitatif. Les quantités y sont données en % en poids.

**Exemple 1**

| Guar éthylée ayant un degré de substitution d'environ 2,5 | 4 % |
|---|---|
| Octyldodécanol | qsp 100 % |

La viscosité est de 2,96 Pa.s (2960 cps). Ce gel transparent peut servir de base à toute composition cosmétique ou dermatologique.

**Exemple 2**

| Guar éthylée ayant un degré de substitution d'environ 2,5 | 4 % |
|---|---|
| Triglycérides d'acides caprique/caprylique | qsp 100 % |

La viscosité est de 2,91 Pa.s (2910 cps). Ce gel transparent peut servir de base à toute composition cosmétique ou dermatologique.

**Exemple 3**

| Guar éthylée ayant un degré de substitution d'environ 2,5 | 4 % |
|---|---|
| Huile de ricin | qsp 100 % |

La viscosité est de 67 Pa.s (67000 cps). Ce gel transparent peut servir de base à toute composition cosmétique ou dermatologique.

**Contre exemple 1**

Avec le $C_{12}$-$C_{15}$ alkylbenzoate dont les paramètres de solubilité $\delta_D$ et $\delta_a$ sont respectivement 17,33 et 4,92, on obtient une composition liquide ayant une viscosité de 0,35 Pa.s (350 cps) pour 4% d'épaississant.

Toutes les compositions ci-dessus sont obtenues en mélangeant l'huile et le polymère en chauffant le mélange à 90 °C jusqu'à dissolution complète du polymère. Le gel est obtenu par refroidissement jusqu'à la température ambiante.

**Exemple 4** : Rouge à lèvres

| | |
|---|---|
| Guar éthylée ayant un degré de substitution d'environ 2,5 | 2,60 % |
| Octyldodécanol | 31,20 % |
| Triglycérides d'acides caprique/caprylique | 31,20 % |
| Polyéthylène (cire) | 15,00 % |
| Lanoline | 10,00 % |
| Pigments | 10,00 % |
| Parfum | qsp |
| Conservateur | qsp |

A partir du gel transparent préparé comme ci-dessus, on ajoute à 90°C la cire de polyéthylène, la lanoline et les pigments. Après broyage des pigments et addition du parfum et du conservateur, on coule le rouge à lèvres dans un moule de forme approprié pour obtenir un bâton.

L'invention est définie pour les revendications.

**Revendications**

1. Composition contenant une phase grasse et un épaississant de la phase grasse, caractérisée en ce que épaississant comprend au moins un alkyléther de polysaccharide formé de motifs comportant au moins deux cycles osidiques différents, chaque motif comportant au moins un groupe hydroxyle substitué par une chaîne alkyle hydrocarbonée saturée et la phase grasse présente les paramètres de solubilité $\delta_D$ et $\delta_a$ selon l'espace de solubilité de Hansen satisfaisant aux conditions suivantes :

$$15,00 \, (\text{J/cm}^3)^{1/2} \leq \delta_D \leq 18,00 \, (\text{J/cm}^3)^{1/2}$$

$$6,50 \, (\text{J/cm}^3)^{1/2} \leq \delta_a \leq 10,00 \, (\text{J/cm}^3)^{1/2}.$$

2. Composition selon la revendication 1, caractérisée en ce que le paramètre de solubilité $\delta_D$ est tel que $16,00 \, (\text{J/cm}^3)^{1/2} \leq \delta_D \leq 18,00 \, (\text{J/cm}^3)^{1/2}$.

3. Composition selon la revendication 1 ou 2, caractérisée en ce que deux à quatre groupes hydroxyle par motif sont substitués par une chaîne alkyle hydrocarbonée saturée.

4. Composition selon l'une des revendications précédents, caractérisée en ce que la chaîne alkyle hydrocarbonée saturée comporte de 1 à 24 atomes de carbone.

5. Composition selon l'une des revendications précédentes, caractérisée en ce que la chaîne alkyle hydrocarbonée saturée comporte de 2 à 10 atomes de carbone.

6. Composition selon l'une des revendications précédentes, caractérisée en ce que la chaîne alkyle est choisie parmi les chaînes méthyle, éthyle, n-propyle, isopropyle, n-butyle, isobutyle, tertiobutyle.

7. Composition selon l'une des revendications précédentes, caractérisée en ce que les cycles osidiques sont choisis parmi le mannose, le galactose, le glucose, le furanose, le rhamnose, l'arabinose.

8. Composition selon l'une des revendications précédentes, caractérisée en ce que l'alkyléther de polysaccharide est un alkyléther d'une gomme choisie parmi la gomme guar, la gomme de caroube, la gomme de karaya, la gomme adragante et leurs mélanges.

9. Composition selon l'une des revendications précédentes, caractérisée en ce que l'alkyléther de polysaccharide est de la gomme guar à chaîne éthyle avec un degré de substitution de 2 à 3.

10. Composition selon l'une des revendications précédentes, caractérisée en ce que l'alkyléther de polysaccharide a un poids moléculaire supérieur à 200 000.

11. Composition selon l'une des revendications précédentes, caractérisée en ce que l'alkyléther de polysaccharide est présent en une quantité allant de 0,5 à 10 % du poids total de la composition.

12. Composition selon l'une des revendications précédentes, caractérisée en ce que l'alkyléther de polysaccharide est présent en une quantité allant de 2 à 8 % du poids total de la composition.

13. Composition selon l'une des revendications précédentes, caractérisée en ce que la phase grasse contient au moins un corps gras satisfaisant aux relations de Hansen choisis parmi les triglycérides d'acides gras de 4 à 10 atomes de carbone, les huiles de synthèse ayant de 12 à 26 atomes de carbone, le dioctanoate de propylène glycol, le diheptanoate de néopentylglycol, le diisononanoate de diéthylèneglycol, l'alcool oléïque, l'isostéaryl lactate l'octylhydroxystéarate, l'hydroxystéarate d'octyldodécyle, le diisostéarylmalate, le citrate de triisocétyle, l'huile de ricin et leurs mélanges.

14. Composition selon la revendication précédente, caractérisée en ce que le corps gras satisfaisant aux relations de Hansen représente jusqu'à 99% de la phase grasse.

15. Composition selon l'une des revendications précédentes, caractérisée en ce qu'elle se présente sous forme de gel huileux, d'émulsion eau-dans-huile ou d'émulsion huile-dans-eau ou de dispersion d'huile dans eau grâce à des vésicules.

16. Composition selon l'une des revendications précédentes, caractérisée en ce qu'elle contient, en outre, au moins un ingrédient choisi parmi les parfums, les conservateurs, les cires, les pigments, les actifs lipophiles.

17. Composition selon l'une des revendications précédentes, caractérisée en ce qu'elle se présente sous forme d'un bâton de rouge à lèvres, de baume à lèvres ou de composition à appliquer sur un film de rouge à lèvres notamment pour en augmenter la brillance.

18. Utilisation d'un alkyléther de polysaccharide formé de motifs comportant au moins deux cycles osidiques différents, chaque motif comportant au moins un groupe hydroxyle substitué par une chaîne alkyle hydrocarbonée saturée, pour épaissir une phase grasse présentant les paramètres de solubilité $\delta_D$ et $\delta_a$ selon l'espace de solubilité de Hansen satisfaisant aux conditions suivantes :

$$15,00 \ (J/cm^3)^{1/2} \le \delta_D \le 18,00 \ (J/cm^3)^{1/2}$$

$$6,50 \ (J/cm^3)^{1/2} \le \delta_a \le 10,00 \ (J/cm^3)^{1/2}.$$

19. Utilisation selon la revendication 18, caractérisée en ce que l'alkyléther de polysaccharide est un alkyléther d'une gomme choisie parmi la gomme guar, la gomme de caroube, la gomme de karaya, la gomme adragante et leurs mélanges.

20. Utilisation cosmétique de la composition selon l'une des revendications 1 à 17 pour soigner et/ou maquiller les lèvres.

21. Utilisation de la composition selon l'une des revendications 1 à 17 pour la préparation d'un onguent destiné à traiter les lèvres.

22. Procédé de traitement cosmétique des lèvres, consistant à appliquer sur les lèvres une composition telle définie aux revendications 1 à 17.

**Claims**

1. Composition containing a fatty phase and a fatty phase thickener, characterized in that this thickener comprises at least one polysaccharide alkyl ether formed of units containing at least two different saccharide rings, each unit containing at least one hydroxyl group substituted with a saturated hydrocarbon alkyl chain, and the fatty phase having solubility parameters $\delta_D$ and $\delta_a$, according to the Hansen solubility space, which satisfy the following conditions:

$$15.00 \ (J/cm^3)^{1/2} \le \delta_D \le 18.00 \ (J/cm^3)^{1/2}$$

$$6.50 \ (J/cm^3)^{1/2} \le \delta_a \le 10.00 \ (J/cm^3)^{1/2}.$$

2. Composition according to Claim 1, characterized in that the solubility parameter $\delta_D$ is such that $16.00 \ (J/cm^3)^{1/2} \le \delta_D \le 18.00 \ (J/cm^3)^{1/2}$.

3. Composition according to Claim 1 or 2, characterized in that two to four hydroxyl groups per unit are substituted with a saturated hydrocarbon alkyl chain.

4. Composition according to one of the preceding claims, characterized in that the saturated hydrocarbon alkyl chain contains from 1 to 24 carbon atoms.

5. Composition according to one of the preceding claims, characterized in that the saturated hydrocarbon alkyl chain contains from 2 to 10 carbon atoms.

6. Composition according to one of the preceding claims, characterized in that the alkyl chain is chosen from methyl, ethyl, n-propyl, isopropyl, n-butyl, isobutyl and tert-butyl chains.

7. Composition according to one of the preceding claims, characterized in that the saccharide rings are chosen from mannose, galactose, glucose, furanose, rhamnose and arabinose.

8. Composition according to one of the preceding claims, characterized in that the polysaccharide alkyl ether is an alkyl ether of a gum chosen from guar gum, carob gum, karaya gum, gum tragacanth and mixtures thereof.

9. Composition according to one of the preceding claims, characterized in that the polysaccharide alkyl ether is guar gum containing an ethyl chain with a degree of substitution from 2 to 3.

10. Composition according to one of the preceding claims, characterized in that the polysaccharide alkyl ether has a molecular weight of greater than 200,000.

11. Composition according to one of the preceding claims, characterized in that the polysaccharide alkyl ether is present in an amount ranging from 0.5 to 10% of the total weight of the composition.

12. Composition according to one of the preceding claims, characterized in that the polysaccharide alkyl ether is present in an amount ranging from 2 to 8% of the total weight of the composition.

13. Composition according to one of the preceding claims, characterized in that the fatty phase contains at least one fatty substance which satisfies the Hansen relationships, chosen from triglycerides of fatty acids of 4 to 10 carbon atoms, synthetic oils having from 12 to 26 carbon atoms, propylene glycol dioctanoate, neopentyl glycol diheptanoate, diethylene glycol diisononanoate, oleyl alcohol, isostearyl lactate, octyl hydroxystearate, octyldodecyl hydroxystearate, diisostearyl malate, triisocetyl citrate, castor oil and mixtures thereof.

14. Composition according to the preceding claim, characterized in that the fatty substance satisfying the Hansen relationships represents up to 99% of the fatty phase.

15. Composition according to one of the preceding claims, characterized in that it is in the form of an oily gel, a water-

EP 0 795 322 B1

in-oil emulsion or oil-in-water emulsion or a dispersion of oil in water by means of vesicles.

16. Composition according to one of the preceding claims, characterized in that it also contains at least one ingredient chosen from fragrances, preserving agents, waxes, pigments and lipophilic active agents.

17. Composition according to one of the preceding claims, characterized in that it is in the form of a stick of lipstick, a lip balm or a composition to be applied to a film of lipstick, in particular to make it shinier.

18. Use of a polysaccharide alkyl ether formed of units containing at least two different saccharide rings, each unit containing at least one hydroxyl group substituted with a saturated hydrocarbon alkyl chain, in order to thicken a fatty phase having solubility parameters $\delta_D$ and $\delta_a$, according to the Hansen solubility space, which satisfy the following conditions:

$$15.00 \, (\text{J/cm}^3)^{1/2} \leq \delta_D \leq 18.00 \, (\text{J/cm}^3)^{1/2}$$

$$6.50 \, (\text{J/cm}^3)^{1/2} \leq \delta_a \leq 10.00 \, (\text{J/cm}^3)^{1/2}.$$

19. Use according to Claim 18, characterized in that the polysaccharide alkyl ether is an alkyl ether of a gum chosen from guar gum, carob gum, karaya gum, gum tragacanth and mixtures thereof.

20. Cosmetic use of the composition according to one of Claims 1 to 17 to care for and/or make up the lips.

21. Use of the composition according to one of Claims 1 to 17 for the preparation of an ointment intended to treat the lips.

22. Process for the cosmetic treatment of the lips, which consists in applying a composition as defined in Claims 1 to 17 to the lips.

**Patentansprüche**

1. Zusammensetzungen, die eine Fettphase und ein Verdickungsmittel für diese Fettphase enthalten, dadurch gekennzeichnet, daß das Verdickungsmittel mindestens einen Polysaccharid-Alkylether enthält, der aus Einheiten aufgebaut ist, die mindestens zwei verschiedene cyclische Zucker enthalten, wobei jede Einheit mindestens eine mit einem gesättigten Alkylrest substituierte Hydroxylgruppe aufweist und wobei die Löslichkeitsparameter $\delta_D$ und $\delta_a$ der Fettphase entsprechend dem Löslichkeitsraum nach Hansen die folgenden Bedingungen erfüllen:

$$15,00 \, (\text{J/cm}^3)^{1/2} \leq \delta_D \leq 18,00 \, (\text{J/cm}^3)^{1/2}$$

$$6,50 \, (\text{J/cm}^3)^{1/2} \leq \delta_a \leq 10,00 \, (\text{J/cm}^3)^{1/2}$$

2. Zusammensetzungen nach Anspruch 1, dadurch gekennzeichnet, daß der Löslichkeitsparameter $\delta_D$ die folgende Bedingung:

$$16,00 \, (\text{J/cm}^3)^{1/2} \leq \delta_D \leq 18,00 \, (\text{J/cm}^3)^{1/2}$$

erfüllt.

3. Zusammensetzungen nach Anspruch 1 oder 2, dadurch gekennzeichnet, daß pro Einheit zwei bis vier Hydroxylgruppen mit einem gesättigten Alkylrest substituiert sind.

4. Zusammensetzungen nach einem der vorherigen Ansprüche, dadurch gekennzeichnet, daß der gesättigte Alkylrest 1 bis 24 Kohlenstoffatome enthält.

5. Zusammensetzungen nach einem der vorherigen Ansprüche, dadurch gekennzeichnet, daß der gesättigte Alkylrest 2 bis 10 Kohlenstoffatome enthält.

6. Zusammensetzungen nach einem der vorherigen Ansprüche, dadurch gekennzeichnet, daß der Alkylrest unter Methyl, Ethyl, n-Propyl, Isopropyl, n-Butyl, Isobutyl und tert-Butyl ausgewählt ist.

8

7. Zusammensetzungen nach einem der vorherigen Ansprüche, dadurch gekennzeichnet, daß die cyclischen Zucker unter Mannose, Galactose, Glucose, Furanose, Rhamnose und Arabinose ausgewählt sind.

8. Zusammensetzungen nach einem der vorherigen Ansprüche, dadurch gekennzeichnet, daß der Polysaccharid-Alkylether ein Alkylether eines Gummis ist, der unter Guargummi, Johannisbrotkernmehl, Karavagummi und Tragantgummi sowie deren Gemischen ausgewählt ist.

9. Zusammensetzungen nach einem der vorherigen Ansprüche, dadurch gekennzeichnet, daß der Polysaccharid-Alkylether ein ethylsubstituiertes Guargummi ist, dessen Substitutionsgrad 2 bis 3 beträgt.

10. Zusammensetzungen nach einem der vorherigen Ansprüche, dadurch gekennzeichnet, daß der Polysaccharid-Alkylether ein Molekulargewicht größer 200 000 aufweist.

11. Zusammensetzungen nach einem der vorherigen Ansprüche, dadurch gekennzeichnet, daß der Polysaccharid-Alkylether in einem Mengenanteil im Bereich von 0,5 bis 10%, bezogen auf das Gesamtgewicht der Zusammensetzung, vorliegt.

12. Zusammensetzungen nach einem der vorherigen Ansprüche, dadurch gekennzeichnet, daß der Polysaccharid-Alkylether in einem Mengenanteil im Bereich von 2 bis 8%, bezogen auf das Gesamtgewicht der Zusammensetzung, vorliegt.

13. Zusammensetzungen nach einem der vorherigen Ansprüche, dadurch gekennzeichnet, daß die Fettphase mindestens ein Fett enthält, das den Hansen-Beziehungen entspricht und das unter den Triglyceriden der Fettsäuren mit 4 bis 10 Kohlenstoffatomen, den synthetischen Ölen mit 12 bis 26 Kohlenstoffatomen, Propylenglykol-dioctanoat, Neopentylglykol-diheptanoat, Diethylenglykol-diisononanoat, Oleylalkohol, Isostearyl-lactat, Octyl-hydroxystearat, Octyldodecyl-hydroxystearat, Diisostearyl-malat, Triisocetyl-citrat und Ricinusöl sowie deren Gemischen ausgewählt ist.

14. Zusammensetzungen nach Anspruch 13, dadurch gekennzeichnet, daß das Fett, das den Hansen-Beziehungen entspricht, bis zu 99% der Fettphase ausmacht.

15. Zusammensetzungen nach einem der vorherigen Ansprüche, dadurch gekennzeichnet, daß sie in Form eines öligen Gels, einer Wasser-in-Öl- oder Öl-in-Wasser-Emulsion oder einer vesikulären Öl-in-Wasser-Dispersion vorliegen.

16. Zusammensetzungen nach einem der vorherigen Ansprüche, dadurch gekennzeichnet, daß sie zusätzlich mindestens einen unter den Parfums, Konservierungsmitteln, Wachsen, Pigmenten und lipophilen Wirkstoffen ausgewählten Inhaltsstoff enthalten.

17. Zusammensetzungen nach einem der vorherigen Ansprüche, dadurch gekennzeichnet, daß sie in Form eines Lippenstifts, eines Lippenbalsams oder einer Zusammensetzung zum Auftragen auf einen Lippenstiftfilm insbesondere zur Erhöhung des Glanzes dieses Lippenstiftfilms vorliegen.

18. Verwendung eines Polysaccharid-Alkylethers, der aus Einheiten aufgebaut ist, die mindestens zwei verschiedene cyclische Zucker enthalten, wobei jede Einheit mindestens eine mit einem gesättigten Alkylrest substituierte Hydroxylgruppe aufweist, zur Verdickung einer Fettphase, deren Löslichkeitsparameter $\delta_D$ und $\delta_a$ entsprechend dem Löslichkeitsraum nach Hansen die folgenden Bedingungen erfüllen:

$$15,00 \; (J/cm^3)^{1/2} \leq \delta_D \leq 18,00 \; (J/cm^3)^{1/2}$$

$$6,50 \; (J/cm^3)^{1/2} \leq \delta_a \leq 10,00 \; (J/cm^3)^{1/2}.$$

19. Verwendung nach Anspruch 18, dadurch gekennzeichnet, daß der Polysaccharid-Alkylether ein Alkylether eines Gummis ist, das unter Guargummi, Johannisbrotkernmehl, Karayagummi und Tragantgummi sowie deren Gemischen ausgewählt ist.

20. Kosmetische Verwendung der Zusammensetzung nach einem der Ansprüche 1 bis 17 zur Lippenpflege und/oder zum Schminken der Lippen.

21. Verwendung der Zusammensetzung nach einem der Ansprüche 1 bis 17 zur Herstellung einer Salbe, die zur Behandlung der Lippen bestimmt ist.

22. Verfahren zur kosmetischen Behandlung der Lippen, das darin besteht, eine Zusammensetzung nach einem der Ansprüche 1 bis 17 auf die Lippen aufzubringen.